# EUROPÄISCHE PATENTSCHRIFT

(11) **EP 0 726 907 B1**
(45) Veröffentlichungstag und Bekanntmachung des Hinweises auf die Patenterteilung: **27.11.2002**
(21) Anmeldenummer: 94928846.8
(22) Anmeldetag: 30.09.1994
(51) Int. Cl.: C07K 1/18, C07K 1/20, C07K 1/22, C07K 14/755, A61K 38/37

(54) **VERFAHREN ZUR HERSTELLUNG EINER VIRUSINAKTIVIERTEN FAKTOR VIII ENTHALTENDEN FRAKTION MITTELS CHROMATOGRAPHISCHER METHODEN**
PROCESS FOR PRODUCING A VIRUS-INACTIVATED FACTOR VIII-CONTAINING FRACTION BY CHROMATOGRAPHIC METHODS
PROCEDE DE PREPARATION D'UNE FRACTION CONTENANT UN FACTEUR VIII A VIRUS INACTIVES AU MOYEN DE METHODES CHROMATOGRAPHIQUES

(30) Priorität: 04.11.1993 DE 4337573
(43) Veröffentlichungstag der Anmeldung: 21.08.1996
(73) Patentinhaber: Octapharma AG, 8853 Lachen (CH)
(72) Erfinder: STRANCAR, Ales, 65270 Ajdovscina (SI); STADLER, Monika, Andrea, A-2435 Wienerherberg (AT); JOSIC, Djuro, A-1020 Wien (AT)
(74) Vertreter: Meyers, Hans-Wilhelm, Dr.Dipl.-Chem.
(86) Internationale Anmeldenummer: EP9403258
(87) Internationale Veröffentlichungsnummer: WO95012609

(56) Entgegenhaltungen:
- EP-A- 0 173 242
- EP-A- 0 233 620
- EP-A- 0 286 323
- EP-A- 0 303 064
- EP-A- 0 567 448
- WO-A-90/05018
- WO-A-90/05140
- US-A- 4 774 323
- JOURNAL OF CHROMATOGRAPHY, Bd.590, Nr.1, 24. Januar 1992, AMSTERDAM Seiten 59 - 76 JOSIC ET AL 'HIGH.PERFORMANCE MEMBRANE CHOMOMATOGRAPHY OF SERUM AND PLASMA MEMBRANE PROTEINS'
- JOURNAL OF CHROMATOGRAPHY, Bd.632, Nr.1-2, 19. Februar 1993, AMSTERDAM Seiten 1 - 10 JOSIC ET AL 'ISOLATION OF PLASMA PROTEINS FROM THE CLOTTING CASCADE BY HEPARIN AFFINITY CHROMATOGRAPHY'

## Beschreibung

Die Erfindung betrifft ein Verfahren zur Herstellung von einer virusinaktivierten Faktor VIII enthaltenden Fraktion mittels chromatographischer Methoden.

Faktor VIII ist ein lebenswichtiger Stoff, der bei der Blutgerinnung eine bedeutende Rolle spielt. So sind Störungen der Blutgerinnung mit der Gabe von Faktor VIII therapierbar. Es besteht daher ein großer Bedarf an verabreichbaren Faktor VIII-Präparationen. Es hat nicht an Versuchen gefehlt, Faktor VIII in stark angereicherter Form aus natürlichen Quellen zu isolieren. So sind bereits chromatographische Methoden bekannt zur Reinigung von Faktor VIII aus Cryopräzipitat. Dieses ist eine Fraktion, die durch Kältebehandlung von Plasma erhältlich ist. Die EP 367 840 B1 betrifft ein chromatographisches Verfahren zur Isolierung von Faktor VIII aus Blutplasma ohne vorherige Cryopräzipitation. Dabei wird die Faktor VIII enthaltende Fraktion durch eine chromatographische Trennung an einem hydrophilen Chromatographiematerial, das mit Ionenaustauschgruppen modifiziert ist, getrennt. Die EP 0 238 701 betrifft ein Verfahren zur Herstellung eines hochreinen, infektiösen Antihämophiliefaktors, wobei die vorbehandelten Fraktionen ein Cryopräzipitat sind, die mittels Ethanolfällung von Fibrinogen, Globulin, Albuminen und anderen störenden Bestandteilen befreit werden. In der europäischen Patentanmeldung EP-A-0 343 275 wird beschrieben, nach Virusinaktivierung der Cryopräzipitatfraktion eine chromatographische Trennung mit Ionenaustauschern durchzuführen. Die EP 0 173 242 A beschreibt ein Verfahren zur chromatographischen Gewinnung von Faktor VIII-Präparationen an Anionenaustauschermaterialien, die ausschließlich auf Kohlenhydraten basieren. Die Kohlenhydratmatrix ist dabei modifiziert mit DEAE-Gruppen. Es werden insbesondere DEAE-Sepharose sowie DEAE-Cellulose als geeignet beschrieben. In der GB-A-1,178,958 wird eine Reinigung des Faktors VIII mit ECTEOLA-Cellulose-Säulen beschrieben. Die modifizierte Cellulose enthält basische Substituenten, die durch Reaktion von Epichlorhydrin und Triethanolamin eingeführt worden sind. Der genannte Stand der Technik nutzt entweder die chromatographische Trennung in Form von Batch-Verfahren oder Säulen-Chromatographie. Mit diesen Verfahren werden zwar recht gute Resultate erzielt, jedoch ist es nach wie vor wünschenswert, die Ausbeute an biologisch wertvollem Faktor VIII sowohl aus wirtschaftlichen wie auch aus ethischen Gründen zu erhöhen.

EP-A-0 286 323 betrifft ein Vefahren zür Reinigüng von Polypeptichen mittels immobilisierter Antikörper. Es werden weitere Reinigüngschrifte an mit Anionenaustaüschern modifizierten Cellulose membranen beschrieben. F VIII lässt sich mit dem diert beschreibenen Verfahren reinigen.

Das der Erfindung zugrundeliegende technische Problem besteht also darin, ein Verfahren bereitzustellen, das ausgehend vom Stand der Technik eine verbesserte Herstellung des Faktors VIII hinsichtlich der Ausbeute und biologischer Aktivität ermöglicht.

Überraschenderweise wird das Problem gelöst durch ein Verfahren zur Herstellung einer virusinaktivierten Faktor VIII enthaltenden Fraktion mittels chromatographischer Methoden, wobei - ausgehend von Cryopräzipitat nach Auflösung oder Blutplasma - gegebenenfalls gefolgt von einer Behandlung mit Aluminiumhydroxid, eine Virusinaktivierung mittels Behandlung der Fraktion mit einem Di- oder Trialkylphosphat und einem nicht-ionischen Tensid erfolgt, anschließend mindestens eine Trennungsoperation mittels Membranchromatographie an Membranen oder kompakten Disks aus den porösen hydrophilen Polymeren Polyglycidylmethacrylaten oder hydrophilisiertem Polystyrol durchgeführt wird.

Das erfindungsgemäße Verfahren läßt sich auch mit handelsüblichem Cryopräzipitat oder Blutplasma durchführen. Vorzugsweise wird zur Vorkonzentrierung des Faktor VIII das aufgetaute Cryopräzipitat zur weiteren Vorreinigung der Probe mit Aluminiumhydroxid behandelt.

Vor der eigentlichen chromatographischen Reinigung an Materialien, die in oder an Membranen angeordnet sind, eine Virusinaktivierung durchgeführt. Die Virusinaktivierung erfolgt dabei gemäß der Methode, die in der EP 131 740 A1 beschrieben wird durch Behandlung mit biokompatiblen organischen Lösungsmitteln (Detergenzien), Triton® X - 100/TNBP vorzugsweise Tween®/TNBP (Tri-n-Butylphosphat). Gute Ergebnisse werden auch mit Natriumcholat/TNBP erzielt. Vorzugsweise werden Mengen von bis zu 15 Gew.-% Detergenz eingesetzt.

Die chromatographische Trennoperation zur Reinigung des Faktors VIII in der Probe können zum einen an mit Ionenaustauschergruppen modifizierten Grundmaterialien, insbesondere Anionenaustauschern, oder mit Immunaffinitätsliganden modifizierten Materialien erfolgen. Entscheidend dabei ist, daß die genannten Materialen in Membranen angeordnet sind. Membranen sowie kompakte Disks aus porösen Polyglycidylmethacrylaten und/oder hyrophilisiertem Polystyrole sind geeignet.

Eine zur Trennung geeignete Membran besteht aus kompakten Scheiben aus Polymerträgern. Die Grundmaterialien der Membranen oder Disks sind mit entsprechenden Anionenaustauschergruppen oder Immunaffinitätsliganden versehen. Als Ionenaustauschergruppen kommen insbesondere Anionenaustauschergruppen wie quaternäre Amine oder Diethylaminoethyl-Gruppen in Frage. Als Kationenaustauscher kommen grundsätzlich schwach und stark saure Kationenaustauscher in Betracht, wie Materialien, die mit Sulfonsäure- oder Phosphorsäuregruppen modifiziert sind.

Die Ionenaustauschergruppen können ohne oder mit einem sogenannten Spacer an die Faser des Grundmaterials gebunden sein. Mit Spacer versehene Materialen werden auch als Tentakelmaterialien bezeichnet. In der DE-A-42 04 694 sind entsprechende Spacer und Liganden genannt. Als Spacer kann auch beispielsweise ein Glucosaminrest dienen. Auch an den Membranen aus porösem Polyglycidylmethacrylat oder den anderen genannten Materialien können Anionenaustauschergruppen wie DEAE oder quaternäre Amine gebunden sein. Die Bindung der Anionenaustauschergruppen erfolgt dabei entweder direkt an das die Membran bildende Material oder ebenfalls über einen Spacer, z.B. einen Glucosaminrest.

In einer anderen Ausführungsform des erfindungsgemäßen Verfahrens wird eine Affinitätsmembranchromatographie mit immobilisierten Substanzen, die eine hohe Affinität für Faktor VIII aufweisen, verwendet.

Die Substanzen mit Affinität für Faktor VIII werden an den Träger mittels chemisch aktiver Gruppen immobilisiert. Vorzugsweise wird die aktive Gruppe nicht direkt am Trägermaterial angreifen, sondern am Ende eines Spacers. Die Immobilisierung der Substanzen für Faktor VIII erfolgt durch Bindung an aktive Gruppen wie Tosyl, Tresyl, Hydrazid und andere. Entsprechende Verfahren sind bekannt aus T.M. Phillips " Affinity chromatography" in "Chromatography" (E. Heftmann, ed.), 5th ed. Elsevier, Amsterdam 1992.

In einer anderen bevorzugten Ausführungsform werden Materialien zur Trennung des Faktors VIII eingesetzt, die eine hydrophobe Wechselwirkung gewährleisten können. Als hydrophobe Materialien werden acyklische und/oder cyklische Alkylketten beispielsweise C₁ bis C₁₈ Alkylketten sowie aromatische Substanzen eingesetzt. Als hydrophobe Wechselwirkung bereitstellende Materialien kommen vorzugsweise auch solche mit abgestufter Hydrophobizität in Frage. Die Hydrophobizität kann durch Einführung von polaren, wie polar-protischen oder polar-a-protischen Gruppen, wie Hydroxy-, Amino-, Cyano-Gruppen abgestuft werden. Vorzugsweise wird dies unter Berücksichtigung der jeweiligen Trennungsbedingungen angepaßt.

Die Virusinaktivierung kann auch durch eine Hitzebehandlung erfolgen. Vorzugsweise wird dabei nach einer ersten Membranchromatographie die Faktor VIII haltige eluierte Probe einem Pasteurisierungsschritt unterzogen. Ein entsprechendes Verfahren wird in der DE-A-43 18 435 vorgeschlagen. Dabei werden Fraktionen, die Faktor VIII angereichert sind, in Gegenwart von Stabilisatoren, mit Di- oder Trialkylphosphaten und gegebenenfalls Benetzungsmitteln in Kontakt gebracht und gleichzeitig oder aufeinanderfolgend bei erhöhter Temperatur im Bereich von 55°C bis 67°C für eine Zeitdauer von 5 Stunden bis 30 Stunden behandelt. Es kann vorteilhaft sein die beiden Methoden der Virusinaktivierung, der Behandlung mit Detergenzien und Hitze zu kombinieren.

Zur Entfernung der bei dem Pasteurisierungsschritt eingesetzten Chemikalien kann auch eine zweite Membranchromatographie angeschlossen werden. Vorzugsweise erfolgt die Trennung der zugegebenen Stabilisatoren mit DEAE- oder quaternären Ammonium-Verbindungen, die über einen Spacer an der Oberfläche des chromatographischen Trägermaterials angeordnet sind, modifizierten Membran. Es ist ebenfalls möglich, die entsprechenden Liganden ohne Spacer an der Oberfläche des Trägermaterials anzuordnen. Die Stabilisatoren werden von diesem Anionenaustauschermaterial unter den gewählten Bedingungen nicht retardiert, während der Faktor VIII an dem Chromatographie-Material adsorbiert wird.

Danach wird der Faktor VIII mit einem wäßrigen Lösungsmittelsystem eluiert mit stufenweise steigenden Salzkonzentrationen.

Die so gewonnene Faktor VIII enthaltende Fraktion wird mittels konventionellen Methoden konzentriert abgefüllt und gegebenenfalls lyophilisiert.

Vorzugsweise wird Faktor VIII im ersten membranchromatographischen Trennungsschritt aus einer Lösung geringer Ionenstärke aufgetragen. Vorzugsweise weist das wäßrige System eine Ionenstärke auf, die einer 0 bis 150 mM Lösung Natriumchlorid entspricht. Bei diesen Ionenstärken ist der Faktor VIII noch am chromatographischen Material adsorbiert, wohingegen schwächer bindende Verunreinigungen durch Auswaschen mit wäßrigen Systemen der gleichen Ionenstärke ausgewaschen werden können.

Eine Reinigung des adsorbierten Materials kann in einer anderen Ausführungsform des erfindungsgemäßen Verfahrens mit einem wäßrigen System mit einer Ionenstärke erfolgen, die einer 200 bis 400 mM Lösung Natriumchlorid entspricht. Die Desorption des Faktor VIII und Elution dieser Fraktion erfolgt dann mit einem wäßrigen System mit einer Ionenstärke, die 500 bis 1500 mM Natriumchlorid-Lösung entspricht. Der pH-Wert wird dabei im Bereich von 4 bis 9 gehalten. Wird eine Kationenaustauscherchromatographie durchgeführt, findet diese vorzugsweise bei einem pH-Wert < 6 statt, wohingegen die Anionenaustauscherchromatographie eher bei höheren pH-Werten über 6 durchgeführt wird.

Wird eine Reinigung des Faktors VIII mit Immunaffinitätsmembranchromatographie durchgeführt, wird im Unterschied zur obengenannten Methode mittels Anionenaustauscher-Materialien die Elution mit chaotropen Reagenzien oder hochkonzentrierten Salzlösungen durchgeführt. Die Elution erfolgt vorzugsweise mit solchen Konzentrationen an chaotropen Reagenzien oder Salzen, die ausreichen, die Bindung zwischen der Substanz mit hoher Affinität für Faktor VIII und Faktor VIII selbst zu lösen. Die Konzentration der genannten Stoffe in den jeweiligen Elutionssystemen hängt dabei von der Stärke der Affinität des Faktors VIII und der entsprechenden bindenden Komponente ab. Dadurch kann eine Elution mit wäßrigen Lösungen geringerer denaturierender Potenz erfolgen. Vorzugsweise werden wäßrige Lösungen mit einer Konzentration von 1 bis 6 M Harnstoff, insbesondere von 2 bis 4 M Harnstoff, oder entsprechend hoch konzentrierten Salzlösungen zur Elution des Faktors VIII von der Immunaffinitätsmembran eingesetzt.

Bei hydrophober Interaktionschromatographie wird die Probe in einer wäßrigen Lösung sehr hoher Ionenstärke aufgetragen, wie beispielsweise hochkonzentriertes Ammoniumsulfat (bis zu einer Konzentration von 4 M) oder Natriumchlorid (bis zu einer Konzentration von bis zu 5 M). Die Elution erfolgt insbesondere in Stufen oder kontinuierlich mit Salzlösungen geringerer Ionenstärke. Als Lösungen mit niedrigerer Ionenstärke zur Elution der Proben bei der hydrophoben Interaktionsmembranchromatographie kann auch eine wäßrige Lösung mit organischen Lösungsmitteln insbesondere eine verdünnte alkoholische Lösung verwendet werden.

Die erfindungsgemäße Verfahrensweise gewährleistet in überraschender Weise eine schnelle und unkomplizierte Reinigung des Faktors VIII, der gleichzeitig in hoher Reinheit und mit hoher Ausbeute anfällt. Darüberhinaus ist die spezifische Aktivität des auf diese Weise gewonnenen Faktors VIII recht hoch, was auf eine geringe Denaturierung des aktiven Faktors durch das erfindungsgemäße Verfahren erklärbar ist.

## Patentansprüche

1. Verfahren zur Herstellung einer virusinaktivierte Faktor VIII enthaltenden Fraktion mittels chromatographischer Methoden, wobei - ausgehend von Cryopräzipitat nach Auflösung oder Blutplasma - gegebenenfalls gefolgt von einer Behandlung mit Aluminiumhydroxid, eine Virusinaktivierung mittels Behandlung der Fraktion mit einem Di- oder Trialkylphosphat und einem nicht-ionischen Tensid erfolgt, anschließend mindestens eine Trennungsoperation mittels Membranchromatographie an Membranen oder kompakten Disks aus den porösen hydrophilen Polymeren Polyglycidylmethacrylaten oder hydrophilisiertem Polystyrol durchgeführt wird.

2. Verfahren nach Anspruch 1, wobei die Trennungsoperation an einem in oder an einer Membran angeordneten Ionenaustauschermaterial, insbesondere Anionenaustauschermaterial erfolgt.

3. Verfahren nach Anspruch 1 und/oder 2, wobei eine weitere Virusinaktivierung durch einen Pasteurisierungsschritt erfolgt, gegebenenfalls gefolgt von einer weiteren Trennungsoperation mittels Membranchromatographie.

4. Verfahren nach mindestens einem der Ansprüche 1 und/oder 3, wobei die Membranchromatographie an einem Material erfolgt, das eine hohe Affinität für Faktor VIII aufweist.

5. Verfahren nach mindestens einem der Ansprüche 1, und/oder 4, wobei das die hohe Affinität für Faktor VIII aufweisende Material mit Liganden mit hohem und/oder niedrigem Molekulargewicht modifiziert ist.

6. Verfahren nach mindestens einem der Ansprüche 4 bis und/oder 5, wobei das eine hohe Affinität für Faktor VIII aufweisende modifizierte Material immobilisierte Liganden mit hoher Affinität für Faktor VIII aufweist.

7. Verfahren nach einem der Ansprüche 1 und/oder 3, wobei das chromatographische Material eine hydrophobe Wechselwirkung mit dem zu trennenden Faktor VIII ermöglicht bzw. entsprechende Liganden aufweist, die eine hydrophobe Wechselwirkung vermitteln.

8. Verfahren nach mindestens einem der Ansprüche 1 bis 3, wobei die zu reinigende Probe in einem wäßrigen System geringer Ionenstärke entsprechend 0 bis 150 mM Natriumchlorid auf die Ionenaustauschermaterial enthaltende Membran aufgetragen, mit einem wäßrigen System höherer Ionenstärke entsprechend 200 bis 400 mM Natriumchloridlösung gegebenenfalls gewaschen und danach mit einem wäßrigen System hoher Ionenstärke entsprechend 500 bis 1.500 mM Natriumchloridlösung eluiert wird, wobei ein pH-Wert von 4 bis 9 eingehalten wird.

9. Verfahren nach mindestens einem der Ansprüche 1, 3 und/oder 7, wobei die zu reinigende Probe aus einer Lösung sehr hoher Ionenstärke auf eine Membran aufgetragen wird, die auf ihre Oberfläche hydrophobe Liganden aufweist und mit Lösungsmittelsystemen geringerer Ionenstärke eluiert wird.

10. Verfahren nach mindestens einem der Ansprüche 1 bis 9, wobei die eluierte Faktor VIII enthaltende Fraktion konzentriert, abgefüllt und/oder lyophilisiert wird.

11. Verfahren nach mindestens einem der Ansprüche 1 bis 10, wobei die Virusinaktivierung durch Behandlung mit bis zu 15 Gew.-% Detergenz erfolgt.

## Claims

1. A process for the preparation of a virus inactivated fraction containing factor VIII by means of chromatographic methods, wherein - proceeding from a cryoprecipitate after dissolution thereof or blood plasma - optionally followed by aluminum hydroxide treatment, a virus inactivation is performed by treatment of said fraction with a di- or trialkyl phosphate and a non-ionic surfactant, followed by at least one separation step using membrane chromatography on membranes or compact disks of the porous hydrophilic polymers poly(glycidyl methacrylates) or hydrophilized polystyrene.

2. The process according to claim 1, wherein said separation step takes place on an ion-exchange material, particularly anion-exchange material, provided within or on a membrane.

3. The process according claim 1 and/or 2, wherein a further virus inactivation is performed by a pasteurization step, optionally followed by an additional separation step using membrane chromatography.

4. The process according to at least one of claims 1 and/or 3, wherein said membrane chromatography takes place on a material having a high affinity for factor VIII.

5. The process according to at least one of claims 1 and/or 4, wherein said material having a high affinity for factor VIII is modified by ligands having high and/or low molecular weights.

6. The process according to at least one of claims 4 and/or 5, wherein said modified material having a high affinity for factor VIII bears immobilized ligands having a high affinity for factor VIII.

7. The process according to any of claims 1 and/or 3, wherein said chromatographic material allows for hydrophobic interaction with the factor VIII to be separated or bears appropriate ligands mediating hydrophobic interaction.

8. The process according to at least one of claims 1 to 3, wherein the sample to be purified is applied to the membrane containing the ion-exchange material in an aqueous system having a low ionic strength corresponding to 0 to 150 mM sodium chloride, optionally washed with an aqueous system having a higher ionic strength corresponding to a 200 to 400 mM sodium chloride solution, followed by elution with an aqueous system having a high ionic strength corresponding to a 500 to 1,500 mM sodium chloride solution, while the pH value is maintained at from 4 to 9.

9. The process according to at least one of claims 1, 3 and/or 7, wherein the sample to be purified is applied from a solution having a very high ionic strength to a membrane bearing hydrophobic ligands on its surface, and is eluted with solvent systems having lower ionic strengths.

10. The process according to at least one of claims 1 to 9, wherein the eluted fraction containing factor VIII is concentrated, filled into appropriate containers and/or lyophilized.

11. The process according to at least one of claims 1 to 10, wherein said virus inactivation is performed by treatment with up to 15% by weight of a detergent.

## Revendications

1. Procédé de préparation par méthodes chromatographiques d'une fraction contenant du facteur VIII inactivé vis à vis des virus, dans lequel - à partir de cryoprécipité après dissolution ou de plasma sanguin - on effectue une inactivation virale au moyen d'un traitement de la fraction avec du di- ou trialkylphosphate et d'un tensioactif non ionique, éventuellement suivi par un traitement avec de l'hydroxyde d'aluminium, et dans lequel on effectue au moins une opération de séparation par chromatographie par membrane sur des membranes ou des disques compacts en les polymères hydrophiles poreux polyglycidylméthacrylate ou polystyrène hydrophylisé.

2. Procédé selon la revendication 1, dans lequel l'opération de séparation s'effectue sur une matière échangeuse d'ions appliquée dans ou sur une membrane, notamment sur une matière échangeuse d'anions.

3. Procédé selon la revendication 1 et/ou 2, dans lequel l'on effectue une inactivation virale complémentaire par pasteurisation, suivie éventuellement d'une autre opération de séparation par chromatographie sur membrane.

4. Procédé selon au moins une des revendications 1 et/ou 3, dans lequel la chromatographie sur membrane s'effectue sur une matière qui présente une grande affinité pour le facteur VIII.

5. Procédé selon au moins une des revendications 1 et/ou 4, dans lequel la matière présentant une grande affinité pour le facteur VIII est modifiée avec des ligands de masse molaire élevée et/ou faible.

6. Procédé selon au moins une des revendications 4 et/ou 5, dans lequel la matière modifiée présentant une grande affinité pour le facteur VIII comporte des ligands fixés ayant une forte affinité pour le facteur VIII.

7. Procédé selon une des revendications 1 et/ou 3, dans lequel le matériau chromatographique permet une interaction hydrophobe avec le facteur VIII à séparer ou comporte des ligands correspondants produisant une interaction hydrophobe.

8. Procédé selon au moins une des revendications de 1 à 3, dans lequel l'échantillon à purifier est appliqué sur une membrane contenant la matière échangeuse d'ions, dans un système aqueux faiblement ionique équivalent à 0-150 mmoles de chlorure de sodium, est éventuellement purifié dans un système aqueux plus ionique, équivalent à une solution de chlorure de sodium de 200 à 400 mmoles, et est ensuite élué par un système aqueux encore plus ionique équivalent à une solution de chlorure de sodium de 500 à 1500 mmoles, une valeur de pH entre 4 et 9 étant observée.

9. Procédé selon au moins une des revendications 1, 3 et/ou 7, dans lequel l'échantillon à purifier est appliqué sous forme d'une solution très fortement ionique sur une membrane comportant des ligands hydrophobes en surface, et est élué par un système de solvants moins ionique.

10. Procédé selon au moins une des revendications de 1 à 9, dans lequel la fraction éluée contenant le facteur VIII est concentrée, soutirée et/ou lyophilisée.

11. Procédé selon au moins une des revendications de 1 à 10, dans lequel l'inactivation virale s'effectue par un traitement avec une solution de détergent d'une concentration allant jusqu'à 15 % en poids.
